# EUROPEAN PATENT SPECIFICATION

(11) **EP 1 937 362 B1**
(45) Date of publication and mention of the grant of the patent: **27.03.2013**
(21) Application number: 06779468.5
(22) Date of filing: 15.09.2006
(51) Int. Cl.: A61K 31/66, A61K 31/663, A61K 31/675, A61K 31/80, A61P 1/04, A61P 19/10

(54) **BISPHOSPHONATE FORMULATION**
BISPHOSPHONAT-FORMULIERUNG
FORMULATION DE BISPHOSPHONATE

(30) Priority: 16.09.2005 GB 0518952; 24.05.2006 GB 0610311
(43) Date of publication of application: 02.07.2008
(73) Proprietor: Selamine Ltd., Dublin (IE)
(72) Inventor: HARRISON, Paul, Dublin 17 (IE); POWER, Anna, Marie, Elizabeth, Dublin 17 (IE)
(74) Representative: Schlich, George William
(86) International application number: PCT/GB2006/003457
(87) International publication number: WO 2007/031785

(56) References cited:
- WO-A-98/14196
- WO-A-2005/115331
- US-A- 5 914 135
- US-A- 6 015 801
- US-A1- 2002 022 603
- US-A1- 2003 206 877
- US-A1- 2004 062 802
- US-A1- 2004 063 670
- SMART H L ET AL: "Comparison of a dimethicone/antacid (Asilone gel) with an alginate/antacid (Gaviscon liquid) in the management of reflux oesophagitis." JOURNAL OF THE ROYAL SOCIETY OF MEDICINE SEP 1990, vol. 83, no. 9, September 1990 (1990-09), pages 554-556, XP009081421 ISSN: 0141-0768

## Description

### Field of the Invention

The present invention relates to formulations comprising bisphosphonates and their use in treatment of various conditions, especially such formulations for treatment of osteoporosis.

### Background to the Invention

Osteoporosis is a disease of bone in which the amount of bone is decreased and the strength of trabecular bone is reduced, cortical bone becomes thin and bones are susceptible to fracture.

It is estimated that 10 million Americans have established osteoporosis and another 34 million have osteopenia, or low bone mass, which leads to osteoporosis. The disease is responsible for 1.5 millions fractures annually, mostly involving the lumbar vertebrae, hip, and wrist.

Patients who are at risk for osteoporosis can be treated with vitamin D and calcium supplements. Bisphosphonates are also commonly used in the prophylaxis and treatment of osteoporosis and corticosteroid-induced osteoporosis. Bisphosphonates are synthetic analogues of natural pyrophosphate that inhibit osteoclast activity and decrease bone turnover and resorption.

The bisphosphonates alendronic acid and risedronate sodium are considered the drugs of choice for treatment of osteoporosis, but disodium etidronate may also be used. Treatment results in lower fracture rates and higher bone density in both male and female patients. Lifestyle changes are also generally prescribed for sufferers.

Whilst it is known to treat osteoporosis with bisphosphonates, there are a number of gastrointestinal symptoms associated with this class of drugs such as abdominal pain, dyspepsia, diarrhoea or constipation. Severe gastrointestinal reactions and oesophageal reactions such as oesophagitis, erosions, and ulceration have occurred. As a consequence biphosphonates should not be administered to patients with abnormalities of the oesophagus or other factors that might delay oesophageal emptying, or those unable to stand, or sit upright for at least 30 minutes (Martindale). Strict instructions are set out for taking these drugs, patients taking alendronate are instructed to take it on an empty stomach before food and to remain sitting upright without eating for at least 30 minutes after taking the drug. Similar instructions, in some case stricter, apply for other bisphosphonates.

The reason for these instructions is that alendronate and other bisphosphonates can provoke severe oesophageal irritation. This can lead to reflux into the oesophagus and consequent ulceration, oesophagitis, heartburn and retrosternal pain, pain on swallowing and dysphagia. In addition to these side-effects, there is reduced patient compliance with the bisphosphonate treatment, leading to progression of the osteoporosis.

Bisphosphonate treatment is so effective that it is very widely used. Patients have hitherto had to put up with the adverse symptoms associated with bisphosphonate use as there is no alternative treatment that gives such good results.

WO 93/09785 and US 2003/0158154 disclose bisphosphonate formulations that contain very small amounts of surfactant, including in some instances simethicone, to facilitate tablet manufacture and give tablets a glossy appearance.

WO 2005/115331 relates to bisphosphonate formulations that comprise a small amount of surfactant in a tablet coating.

US 2002/0022603 relates to bisphosphonate formulations that comprise a zwitterionic phospholipid.

A composition comprising pamidronate and Antifoam® AF is known from US 2004/0063670

US 5,914,135 relates to antacid formulations comprising a pH adjusting agent and optionally simethicone.

Smart et al (Journal of the Royal Society of Medicine, 83:554-556, 1990) relates to formulations comprising an antacid and either dimethicone or alginate.

An object of the invention is to ameliorate the above problems and disadvantages. An object of a specific embodiment of the invention is to provide a formulation of a bisphosphonate which provokes reduced gastric irritation and/or reduced reflux of stomach acid, leading preferably to increased patient compliance.

### Summary of the Invention

Accordingly, the present invention provides a formulation for use in the treatment of osteoporosis, comprising a bisphosphonate in combination with an amount of an antifoaming agent effective to reduce foam formation in a patient's stomach, wherein the amount of antifoaming agent is in the range of 20 mg to 150 mg.

In use, for example in treatment of osteoporosis, enough antifoaming agent is present for the combination to be expected to limit the formation of foams in the stomach. Without wishing to be bound by any particular theory, it is believed that the associated concomitant decrease in the volume of stomach contents, and additionally barrier properties of some preferred antifoaming agents, will reduce the likelihood of stomach acid reflux and therefore oesophageal irritation. Hence, typical formulations of the invention comprise an amount of an antifoaming agent effective to reduce the formation of foam in the stomach.

The antifoaming agent may comprise an agent to lower surface tension and/or to reduce the foaming tendency of stomach contents, and more than one agent may be advantageously used in concert to reduce foaming, and, in preferred embodiments also provide barrier protection.

Formulations of the present invention may comprise an amount of an antifoaming agent in the range of 20 mg to 150 mg, preferably from 35mg to 125mg and specific embodiments set out in examples below have antifoaming agent present in the range of 50 mg to 100 mg - according to the United States Pharmacopeia, the minimum quantity of simethicone effective for reducing foam formation in the stomach is 20 mg. The invention also provides formulations that comprise at least 1%, preferably at least 2% antifoaming agent, and more generally an amount of an antifoaming agent in the range of 3% to 40% by weight, and preferably in the range of 5% to 30% by weight. Specific embodiments set out in the examples contain from about 7% to about 18% antifoaming agent by weight.

Antifoaming agents are known to those of skill in the art. Whilst many different agents may be used in the formulations of the invention, presently there are only a limited number of approved antifoaming agents available for pharmaceutical formulations, and these are particularly suitable. Siloxanes can be used. Some embodiments use one or more polydimethylsiloxanes as the antifoaming agent. Preferred embodiments of the formulation of the invention comprise dimethicone BP, simethicone BP (an activated form of dimethicone), or both.

Any bisphosphonate having the side-effect of promoting gastric irritation may suitably be used in the formulations of the invention. The invention applies generally to formulations of bisphosphonates, including for example alendronic acid, disodium etidronate, disodium pamidronate, ibandronic acid, risedronate sodium, sodium clodronate, strontium ranelate, tiludronic acid and zoledronic acid. In preferred embodiments the bisphosphonate may be selected from the group alendronic acid or alendronate, risedronate and etidronate. Particularly preferred formulations comprise alendronic acid or alendronate. Typically the amount of bisphosphonate is from 5mg to 150mg of Alendronic acid (or a therapeutically equivalent amount of another bisphosphonate, or an equivalent amount of a bisphosphonate compound), preferably from about 10mg to about 70mg. Formulations of the invention comprise also a pharmaceutically acceptable carrier.

Bisphosphonates can be co-administered with other agents helpful in treatment of osteoporosis, either directly or dealing e.g. with side effects of the treatment. Formulations of the invention may thus also include one or more of a vitamin D derivative and a calcium supplement.

The term 'Vitamin D derivative' is used for a range of compounds which have the ability to prevent or treat rickets. Vitamin D supplements suitable for inclusion in formulations of the invention include ergocalciferol (calciferol, vitamin D2), cholecalciferol (vitamin D3), dihydrotachysterol, alfacalcidol (1α-hydroxycholecalciferol), and calcitriol (1,25-dihydroxycholecalciferol).

Some calcium supplements which may be used in the formulations of the invention are calcium salts, optionally selected from calcium gluconate, calcium chloride, calcium lactate, ADCAL®, CACIT®, CALCICHEW®, CALCIUM-500®, CALCIUM-SANDOZ® and SANDOCAL®. Treatments of the invention may also be carried out in combination with parenteral calcium supplements.

Particularly preferred formulations of the invention comprise a bisphosphonate, antifoaming agent, a vitamin D derivative and a calcium supplement.

Formulations of the invention include an amount of one or more agents effective for reducing the tendency of the stomach contents to foam, and which may also elicit barrier protection. Treatment according to the invention involves the administration of one or more such agents with bisphosphonates simultaneously, either together (in the same formulation) or separately (taken together at the same time), or separately (time delayed administration).

Hence, the bisphosphonate and the antifoaming agent can be taken separately. In these embodiments the antifoaming agent is generally taken up to 1 hour before and not more than 10 minutes after the bisphosphonate. Preferably the antifoaming agent is taken not more than 10 minutes before and more preferably not more than 5 minutes before the bisphosphonate.

In a further aspect, the invention provides a kit comprising a bisphosphonate for use in the treatment of osteoporosis and an amount of an antifoaming agent effective to reduce foam formation in a patient's stomach, wherein the amount of antifoaming agent is in the range of 20 mg to 150 mg. Preferred kits include other actives such as vitamin D derivatives and/or calcium supplements.

In a further aspect, the invention provides for the use of an antifoaming agent in the manufacture of a medicament for the treatment or prophylaxis of osteoporosis in combination with a bisphosphonate, wherein the antifoaming agent is present in an amount effective to reduce foam formation in a patient's stomach, and wherein the amount of antifoaming agent is in the range of 20 mg to 150 mg. The invention also provides for the use of a bisphosphonate in the manufacture of a medicament for the treatment or prophylaxis of osteoporosis in combination with an amount of an antifoaming agent effective to reduce foam formation in a patient's stomach, wherein the amount of antifoaming agent is in the range of 20 mg to 150 mg. Preferably the bisphosphonate is alendronate and thee antifoaming agent is siloxane. The medicament may advantageously contain other actives as outlined herein.

Specific embodiments of the invention provide formulations for use in the treatment of osteoporosis, comprising alendronate, an amount of simethicone or dimethicone effective for reducing the formation of foam in the stomach, and a pharmaceutically acceptable carrier, wherein the amount of simethicone or dimethicone is in the range of 20 mg to 150 mg.

For the purposes of the present specification the phrase 'treatment or prophylaxis of osteoporosis' means any and all treatment or prophylaxis for Paget's disease, osteopenia, osteoporosis and corticosteroid-induced osteoporosis.

The invention also provides methods for the treatment of osteoporosis comprising administration to a patient of a bisphosphonate and an antifoaming agent.

The formulations, methods, kits and uses discussed offer potential reduced mucosal irritation, gastric irritation and / or oesophageal irritation when compared to the art known bisphosphonate formulations. An anticipated advantage of the invention is hence that this irritation is reduced. Further anticipated advantages are that instructions to patients, which hitherto gave strict advice as to how to take the medicament, can be relaxed and lower irritation will naturally lead to greater patient compliance with the medication regime and greater overall effectiveness of treatment.

### Examples

Various aspects of the invention will now be described with reference to the accompanying Examples. The examples are not to be construed as limiting the scope of the invention as claimed herein. One of skill in the pharmaceutical arts will understand that some of the ingredients of the formulations given in the examples may be substituted with known equivalent ingredients. These formulations comprise a bisphosphonate for treatment of osteoporosis in combination with an amount of an antifoaming agent effective for reducing foam formation in the stomach, and are within the scope of the invention.

### Example 1

| | |
|---|---|
| Alendronic acid 70mg (as sodium alendronate trihydrate) | 91.4 mg |
| Dimeticone B.P. | 50.0 mg |
| Microcrystalline cellulose B.P. | 140.0 mg |
| Lactose B.P. | 110.0mg |
| Croscarmellose sodium B.P. | 6.0 mg |
| Dehydrated Alcohol USP | q.s. |
| Colloidal anhydrous silica B.P. | 1.0 mg |
| Magnesium stearate | 2.0 mg |
| Total | 400.4mg |

### Example 2

| | |
|---|---|
| Alendronic acid 70mg (as sodium alendronate trihydrate) | 91.4 mg |
| Simeticone B.P. | 50.0 mg |
| Microcrystalline cellulose B.P. | 140.0 mg |
| Calcium carbonate B.P. | 105.0 mg |
| Povidone B.P | 6.0 mg |
| Croscarmellose sodium B.P. | 5.0 mg. |
| Dehydrated Alcohol USP | q.s |
| Colloidal anhydrous silica B.P. | 1.0 mg |
| Magnesium stearate | 2.0 mg |
| Total | 400.4 mg |

### Example 3

| | |
|---|---|
| Alendronic acid 70mg (as sodium alendronate trihydrate) | 91.4 mg |
| Simeticone B.P. | 25.0 mg |
| Dimethicone B.P. | 25.0 mg |
| Microcrystalline cellulose B.P. | 140.0 mg |
| Magnesium carbonate B.P. | 110.0 mg |
| Croscarmellose sodium B.P. | 6.0 mg |
| Dehydrated Alcohol USP | q.s. |
| Colloidal anhydrous silica B.P. | 1.0 mg |
| Magnesium stearate | 2.0 mg |
| Total | 400.4 mg |

### Example 4

| | |
|---|---|
| Alendronic acid 70mg (as sodium alendronate trihydrate) | 91.4 mg |
| Dimeticone B.P. | 50.0 mg |
| Microcrystalline cellulose B.P. | 140.0 mg |
| Sucrose B.P. | 110.0 mg |
| Croscarmellose sodium B.P. | 6.0 mg |
| Dehydrated Alcohol USP | q.s. |
| Colloidal anhydrous silica B.P. | 1.0 mg |
| Magnesium stearate | 2.0 mg |
| Total | 400.4 mg |

### Example 5

| | |
|---|---|
| Alendronic acid 70mg (as sodium alendronate trihydrate) | 91.4 mg |
| Dimeticone B.P. | 50.0 mg |
| Microcrystalline cellulose B.P. | 140.0 mg |
| Lactose B.P. | 110.0 mg |
| Croscarmellose sodium B.P. | 6.0 mg |
| Isopropyl Alcohol USP | q.s. |
| Colloidal anhydrous silica B.P. | 1.0 mg |
| Magnesium stearate | 2.0 mg |
| Total | 400.4 mg |

### Example 6

| | |
|---|---|
| Alendronic acid 70mg (as sodium alendronate trihydrate) | 91.4 mg |
| Dimeticone B.P. | 50.0 mg |
| Microcrystalline cellulose B.P. | 140.0 mg |
| Lactose B.P. | 110.0 mg |
| Croscarmellose sodium B.P. | 6.0 mg |
| Purified Water | q.s. |
| Colloidal anhydrous silica B.P. | 1.0 mg |
| Magnesium stearate | 2.0 mg |
| Total | 400.4 mg |

### Example 7

| | |
|---|---|
| Alendronic acid 70mg (as sodium alendronate trihydrate) | 91.4 mg |
| Dimeticone B.P. | 50.0 mg |
| Microcrystalline cellulose B.P. | 140.0 mg |
| Lactose B.P. | 110.0 mg |
| Croscarmellose sodium B.P. | 6.0 mg |
| Dehydrated Alcohol USP | q.s. |
| Colloidal anhydrous silica B.P. | 1.0 mg |
| Stearic Acid | 2.0 mg |
| Total | 400.4 mg |

### Example 8

| | |
|---|---|
| Alendronic acid 70mg (as sodium alendronate trihydrate) | 91.4 mg |
| Dimeticone B.P. | 100.0 mg |
| Microcrystalline cellulose B.P. | 280.0 mg |
| Lactose B.P. | 113.0 mg |
| Croscarmellose sodium B.P. | 12.0 mg |
| Dehydrated Alcohol USP | q.s. |
| Colloidal anhydrous silica BP | 2.0 mg |
| Magnesium stearate | 3.0 mg |
| Total | 601.4 mg |

### Example 9

| | |
|---|---|
| Alendronic acid 70mg (as sodium alendronate trihydrate) | 91.4 mg |
| Simeticone B.P. | 100.0 mg |
| Microcrystalline cellulose B.P. | 280.0 mg |
| Lactose B.P. | 113.0 mg |
| Croscarmellose sodium B.P. | 12.0 mg |
| Dehydrated Alcohol USP | q.s. |
| Colloidal anhydrous silica BP | 2.0 mg |
| Magnesium stearate | 3.0 mg |
| Total | 601.4 mg |

### Example 10

| | |
|---|---|
| Alendronic acid 70mg (as sodium alendronate trihydrate) | 91.4 mg |
| Dimeticone B.P. | 50.0 mg |
| Simeticone B.P. | 50.0 mg |
| Microcrystalline cellulose B.P. | 280.0 mg |
| Lactose B.P. | 113.0 mg |
| Croscarmellose sodium B.P. | 12.0 mg |
| Dehydrated Alcohol USP | q.s. |
| Colloidal anhydrous silica BP | 2.0 mg |
| Magnesium stearate | 3.0 mg |
| Total | 601.4 mg |

### Example 11

| | |
|---|---|
| Alendronic acid 70mg (as sodium alendronate trihydrate) | 91.4 mg |
| Dimeticone B.P. | 100.0 mg |
| Microcrystalline cellulose B.P. | 280.0 mg |
| Sucrose B.P. | 113.0 mg |
| Croscarmellose sodium B.P. | 12.0 mg |
| Dehydrated Alcohol USP | q.s. |
| Colloidal anhydrous silica BP | 2.0 mg |
| Magnesium stearate | 3.0 mg |
| Total | 601.4 mg |

### Example 12

| | |
|---|---|
| Alendronic acid 70mg (as sodium alendronate trihydrate) | 91.4 mg |
| Dimeticone B.P. | 100.0 mg |
| Microcrystalline cellulose B.P. | 280.0 mg |
| Lactose B.P. | 113.0 mg |
| Croscarmellose sodium B.P. | 12.0 mg |
| Isopropyl alchol B.P. | q.s. |
| Colloidal anhydrous silica BP | 2.0 mg |
| Magnesium stearate | 3.0 mg |
| Total | 601.4 mg |

### Example 13

| | |
|---|---|
| Alendronic acid 70mg (as sodium alendronate trihydrate) | 91.4 mg |
| Dimeticone B.P. | 100.0 mg |
| Microcrystalline cellulose B.P. | 280.0 mg |
| Lactose B.P. | 113.0 mg |
| Croscarmellose sodium B.P. | 12.0 mg |
| Purified water B.P. | q.s. |
| Colloidal anhydrous silica BP | 2.0 mg |
| Magnesium stearate | 3.0 mg |
| Total | 601.4 mg |

### Example 14

| | |
|---|---|
| Alendronic acid 70mg (as sodium alendronate trihydrate) | 91.4 mg |
| Dimeticone B.P. | 100.0 mg |
| Microcrystalline cellulose B.P. | 280.0 mg |
| Lactose B.P. | 113.0 mg |
| Croscarmellose sodium B.P. | 12.0 mg |
| Dehydrated Alcohol USP | q.s. |
| Colloidal anhydrous silica BP | 2.0 mg |
| Stearic acid | 3.0 mg |
| Total | 601.4 mg |

### Example 15

| | |
|---|---|
| Alendronic acid 10mg (as sodium alendronate trihydrate) | 13.1 mg |
| Dimeticone B.P. | 50.0 mg |
| Microcrystalline cellulose B.P. | 140.0 mg |
| Lactose B.P. | 80.0 mg |
| Croscarmellose sodium B.P. | 6.0 mg |
| Dehydrated Alcohol USP | q.s |
| Colloidal anhydrous silica BP | 1.0 mg |
| Magnesium stearate | 2.0 mg |
| Total | 292.1 mg |

### Example 16

| | |
|---|---|
| Alendronic acid 10mg (as sodium alendronate trihydrate) | 13.1 mg |
| Simeticone B.P. | 50.0 mg |
| Microcrystalline cellulose B.P. | mg |
| Lactose B.P. | 80.0 mg |
| Croscarmellose sodium B.P. | 6.0 mg |
| Dehydrated Alcohol USP | q.s |
| Colloidal anhydrous silica BP | 1.0 mg |
| Magnesium stearate | 2.0 mg |
| Total | 292.1 mg |

### Example 17

| | |
|---|---|
| Alendronic acid 10mg (as sodium alendronate trihydrate) | 13.1 mg |
| Simeticone B.P. | 25.0 mg |
| Dimeticone B.P. | 250.0 mg |
| Microcrystalline cellulose B.P. | 140.0 mg |
| Lactose B.P. | 80.0 mg |
| Croscarmellose sodium B.P. | 6.0 mg |
| Dehydrated Alcohol USP | q.s |
| Colloidal anhydrous silica BP | 1.0 mg |
| Magnesium stearate | 2.0 mg |
| Total | 292.1 mg |

### Example 18

| | |
|---|---|
| Alendronic acid 10mg (as sodium alendronate trihydrate) | 13.1 mg |
| Dimeticone B.P. | 50.0 mg |
| Microcrystalline cellulose B.P. | 140.0 mg |
| Sucrose B.P. | 80.0 mg |
| Croscarmellose sodium B.P. | 6.0 mg |
| Dehydrated Alcohol USP | q.s |
| Colloidal anhydrous silica BP | 1.0 mg |
| Magnesium stearate | 2.0 mg |
| Total | 292.1 mg |

### Example 19

| | |
|---|---|
| Alendronic acid 10mg (as sodium alendronate trihydrate) | 13.1 mg |
| Dimeticone B.P. | 50.0 mg |
| Microcrystalline cellulose B.P. | 140.0 mg |
| Lactose B.P. | 80.0 mg |
| Croscarmellose sodium B.P. | 6.0 mg |
| Isopropyl Alcohol USP | q.s |
| Colloidal anhydrous silica BP | 1.0 mg |
| Magnesium stearate | 2.0 mg |
| Total | 292.1 mg |

### Example 20

| | |
|---|---|
| Alendronic acid 10mg (as sodium alendronate trihydrate) | 13.1 mg |
| Dimeticone B.P. | 50.0 mg |
| Microcrystalline cellulose B.P. | 140.0 mg |
| Lactose B.P. | 80.0 mg |
| Croscarmellose sodium B.P. | 6.0 mg |
| Purified water | q.s |
| Colloidal anhydrous silica BP | 1.0 mg |
| Magnesium stearate | 2.0 mg |
| Total | 292.1 mg |

### Example 21

| | |
|---|---|
| Alendronic acid 10mg (as sodium alendronate trihydrate) | 13.1 mg |
| Dimeticone B.P. | 50.0 mg |
| Microcrystalline cellulose B.P. | 140.0 mg |
| Lactose B.P. | 80.0 mg |
| Croscarmellose sodium B.P. | 6.0 mg |
| Dehydrated Alcohol USP | q.s |
| Colloidal anhydrous silica BP | 1.0 mg |
| Stearic Acid | 2.0 mg |
| Total | 292.1 mg |

The formulations of Examples 1-21 are manufactured according to the following manufacturing examples:

### Example 22

To manufacture tablet formulations of the above examples the dry ingredients 1-5 (dry ingredients 1-6 in examples 3, 10 and 17 where both simeticone and dimeticone are included) are mixed together. Item 6 (item 7 for examples 3, 10 and 17) is then added to form a wet granulate suitable for compression. The wet granulate is then dried and milled to give a uniform granule. The dry milled granules are then mixed with excipients 7 & 8 and compressed to a suitable hardness.

### Example 23

Tablets are prepared as above (example 22) but item 2 is omitted from the dry mix and then added to the solvent, ie item 6 (or 7), prior to it being added to the dry mix of ingredient. The remaining steps are the same as for example 22.

### Example 24

Tablets are prepared as above for example 22 but item 6 (or 7), the solvent of granulation, is omitted and the product is manufactured by direct compression.

### Example 25

Ingredients 1-5 (or 1-6) are dry mixed followed by dry milling. The remaining excipients are then blended into the dry ingredients and the resultant mass is compressed to a suitable hardness to give tablets or encapsulated into a suitably sized capsule.

### Example 26

A tablet is made in which simeticone granules are manufactured separately and then blended with the other ingredients.

| | | |
|---|---|---|
| 1. | Simeticone B.P. | 100.0 mg |
| 2. | Mannitol B.P. | 400.0 mg |
| 3. | Povidone B.P. | 6.0 mg |
| 4. | Sodium starch glycollate B.P. | 12.0 mg |
| 5. | Purified Water B.P | q.s |
| | sub total | 518.0 mg |

To make the simeticone granules items 1-4 are dry mixed, water is added and then the mixture is wet mixed. The mixture is then dried and milled to give a uniform granule.

| | | |
|---|---|---|
| A. | Alendronic acid 70mg (as sodium alendronate trihydrate) | 91.4 mg |
| B. | Simethicone granule | 259.0 mg |
| C. | Purified Talc B.P. | 18.0 mg |
| D. | Stearic Acid B.P. | 6.0 mg |
| | Total | 374.4 mg |

The required amount of simeticone granules (in this particular case 259.0 mg) are then mixed with ingredients A, C and D until a uniform mixture is obtained. The mixture is then compressed to a suitable hardness to give tablets.

### Example 27

Tablets are prepared as for the method of Example 26 except that the amounts of items B-D are doubled to give a tablet containing 100mg simeticone with a tablet compression weight of 633.36 mg.

### Example 28

Tablets are prepared as for the method of Example 26 except initially using half amounts of ingredients C & D before dry granulation. The compacted granule is then milled and the remainder of items C & D added. The final mix is then blended and compressed to a suitable hardness to give tablets.

### Example 29

| | | |
|---|---|---|
| 1. | Etidronate sodium | 200.0 mg |
| 2. | Simeticone B.P. | 50.0 mg |
| 3. | Maize starch B.P. | 20.0 mg |
| 4. | Microcrystalline cellulose | 100.0 mg |
| 5. | Purified water | q.s |
| 6. | Maize starch | 30.0 mg |
| 7. | Colloidal anhydrous silica | 1.5 mg |
| 8. | Magnesium stearate | 7.0 mg |
| | Total | 408.5 mg |

Items 1 & 4 are dry mixed and then item 2 is slowly added until dispersed. A small quantity of water is then added followed by the etidronate disodium. The ingredients are mixed until well dispersed and additional water is added to form a wet granule suitable for compression. The wet granule is then dried and milled and the remaining items are added. The mixture is then compressed at a suitable hardness or encapsulate into a size 1 capsule.

### Example 30

Tablets are prepared as for example 26 but alendronate is separately replaced with (a) 35mg of risedronic acid, (b) 400 mg of sodium clodronate, and (c) 200 mg of tiludronic acid.

### Examples 31 - 41

| Ingredient (mg) | 31 | 32 | 33 | 34 |
|---|---|---|---|---|
| Biphosphonate⁺ | Q.S | Q.S | Q.S | Q.S |
| Simethicone USP | | 55.0 | 110.0 | 55.0 |
| Dimethicone NF | 50.0 | - | | - |
| Mannitol | 300.0 | 270.0 | 320.0 | 300.0 |
| Polyvidone | 3.5 | 3.5 | 14.0 | - |
| Maize Starch | 8.0 | | - | |
| Pregelatinised Starch | - | 25.0 | 70.0 | 40.0 |
| Microcrystalline cellulose | 130.0 | 115.0 | 120.0 | 80.0 |
| L-HPC | 20.0 | 32.0 | 30.0 | 30.0 |
| Sodium croscarmellose | 5.0 | 8.0 | 18.0 | - |
| Silicon dioxide | 5.0 | - | - | |
| Sodium stearyl fumarate | - | - | | 5.0 |
| Total | 521.5 mg | 508.5 mg | 682.0 mg | 510.0 mg |

| Ingredient (mg) | 35 | 36 | 37 | 38 |
|---|---|---|---|---|
| Biphosphonate⁺ | q.s | q.s | q.s | q.s |
| Simethicone USP | | | 55.0 | 55.0 |
| Dimethicone NF | 50.0 | 100.0 | | - |
| Lactose hydrous | 300.0 | 360.0 | 300.0 | - |
| Lactose anhydrous | | - | | - |
| Mannitol | | - | | 250.0 |
| Polyvidone | 3.5 | - | 20.0 | - |
| Gelatin. | - | 18.0 | | |
| Maize Starch | 8.0 | 60.0 | - | 7.0 |
| Pregelatinised Starch | - | - | - | - |
| Microcrystalline cellulose | 120.0 | 100.0 | 100.0 | 125.0 |
| L-HPC | 20.0 | 20.0 | 30.0 | 30.0 |
| Sodium croscarmellose Ph.Eur | - | 10.0 | 18.0 | 15.0 |
| Crospovidone | 10.0 | | - | - |
| Silicon dioxide | 5.0 | 10.0 | - | |
| Sodium stearyl fumarate | - | 5.0 | | 5.0 |
| Total | 516.5 mg | 683.0 mg | 523.0 mg | 487.0 mg |

| | | | | |
|---|---|---|---|---|
| ⁺ The therapeutic dose of the biphosphonate. | | | | |

### Capsules

| Ingredient (mg) | 39 | 40 | 41 |
|---|---|---|---|
| Biphosphonate⁺ | q.s | q.s | q.s |
| Simethicone^{*} USP | 110.0 | 55.0 | 110.0 |
| Lactose hydrous | | | 180.0 |
| Lactose anhydrous | | | |
| Mannitol | 200.0 | 150.0 | |
| Gelatin. | - | - | 15.0 |
| Maize Starch | 20.0 | | |
| Pregelatinised Starch | - | 20.0 | |
| Microcrystalline cellulose | 30.0 | 65.0 | 30.0 |
| L-HPC | 10.0 | 10.0 | |
| Sodium croscarmellose Ph.Eur | 5.0 | 5.0 | 15.0 |
| Crospovidone | | - | 10.0 |
| Total | 375.0 mg | 305 mg | 360.0 mg |

The invention thus provides formulations and uses thereof for treatment of osteoporosis with reduced gastric and other irritation.

Although the invention has been described with reference to specific examples one of skill in the art will appreciate that variations may be made to these formulations without departing from the scope of the following claims.

## Claims

1. A formulation for use in the treatment of osteoporosis, comprising a bisphosphonate in combination with an amount of an antifoaming agent effective to reduce foam formation in a patient's stomach, wherein the amount of antifoaming agent is in the range of 20 mg to 150 mg.

2. The formulation of claim 1, wherein the amount of antifoaming agent is in the range of 35 mg to 125 mg for use according to claim 1.

3. The formulation of claim 1, wherein the amount of antifoaming agent is in the range of 3% to 40% by weight for use according to claim 1.

4. The formulation of claim 1, wherein the amount of antifoaming agent is in the range of 5% to 30% by weight for use according to claim 1.

5. The formulation of any of claims 1 to 4, wherein the antifoaming agent is one or more polydimethylsiloxanes for use according to claim 1.

6. The formulation of claim 5 wherein the antifoaming agent is dimethicone BP for use according to claim 1.

7. The formulation of claim 5 wherein the antifoaming agent is simethicone BP for use according to claim 1.

8. The formulation of claim 5 wherein the antifoaming agent comprises dimethicone BP and simethicone BP for use according to claim 1.

9. The formulation of any preceding claim wherein the formulation additionally includes an antacid for use according to claim 1.

10. The formulation of claim 9 wherein the antacid is selected from the group consisting of a carbonate salt, a trisilicate, and an oxide salt for use according to claim 1.

11. The formulation of any preceding claim wherein the bisphosphonate is selected from the group consisting of alendronic acid, disodium etidronate, disodium pamidronate, ibandronic acid, risedronate sodium, sodium clodronate, strontium ranelate, tiludronic acid and zoledronic acid for use according to claim 1.

12. The formulation of any preceding claim wherein the bisphosphonate is selected from the group consisting of alendronic acid, alendronate, risedronate and etidronate for use according to claim 1.

13. The formulation of any preceding claim wherein the bisphosphonate is selected from the group consisting of alendronic acid and alendronate for use according to claim 1.

14. The formulation of any preceding claim additionally comprising (i) a vitamin D derivative, (ii) a calcium supplement or (iii) both (i) and (ii) for use according to claim 1.

15. The formulation of claim 14 wherein the vitamin D derivative is selected from the group consisting of ergocalciferol (calciferol, vitamin D2), cholecalciferol (vitamin D3), dihydrotachysterol, alfacalcidol (1α-hydroxycholecalciferol), and calcitriol (1,25-dihydroxycholecalciferol) for use according to claim 1.

16. The formulation of claim 14 wherein the calcium supplement is selected from the group consisting of calcium salts, calcium gluconate, calcium chloride, calcium lactate, ADCAL®, CACIT®, CALCICHEW®, CALCIUM-500®, CALCIUM-SANDOZ® and SANDOCAL® for use according to claim 1.

17. The formulation of any preceding claim wherein the formulation is formulated for simultaneous administration of actives, either together (in the same formulation) or separately (taken together at the same time), or separately (time delayed administration) for use according to claim 1.

18. The formulation of any preceding claim, comprising a pharmaceutically acceptable carrier for use according to claim 1.

19. A kit comprising a bisphosphonate for use in the treatment of osteoporosis and an amount of an antifoaming agent effective to reduce foam formation in a patient's stomach, wherein the amount of antifoaming agent is in the range of 20 mg to 150 mg.

20. The kit of claim 19, wherein the amount of antifoaming agent is in the range of 3% to 20% by weight for use according to claim 19.

21. The kit of claim 19 or claim 20 additionally comprising one or more vitamin D derivatives for use according to claim 19.

22. The kit of claims 19 to 21 comprising one or more calcium supplements for use according to claim 19.

23. The use of an antifoaming agent in the manufacture of a medicament for the treatment or prophylaxis of osteoporosis in combination with a bisphosphonate, wherein the antifoaming agent is present in an amount effective to reduce foam formation in a patient's stomach, and wherein the amount of antifoaming agent is in the range of 20 mg to 150 mg.

24. The use of a bisphosphonate in the manufacture of a medicament for the treatment or prophylaxis of osteoporosis in combination with an amount of an antifoaming agent effective to reduce foam formation in a patient's stomach, wherein the amount of antifoaming agent is in the range of 20 mg to 150 mg.

25. The use of claim 23 or claim 24 wherein the amount of an antifoaming agent is in the range of 3% to 20% by weight.

26. The use of claims 23 to 25 wherein the medicament additional includes one or more of vitamin D derivatives and calcium supplements.

27. A formulation for use in the treatment of osteoporosis, comprising alendronate, an amount of simethicone effective for reducing the formation of foam in the stomach, and a pharmaceutically acceptable carrier, wherein the amount of simethicone is in the range of 20 mg to 150 mg.

28. A formulation for use in the treatment of osteoporosis, comprising alendronate, an amount of dimethicone effective for reducing the formation of foam in the stomach, and a pharmaceutically acceptable carrier, wherein the amount of dimethicone is in the range of 20 mg to 150 mg.

## Patentansprüche

1. Formulierung zur Verwendung bei der Behandlung von Osteoporose, die ein Bisphosphonat in Kombination mit einer Menge eines Entschäumungsmittels umfasst, die wirksam ist, um Schaumbildung im Magen eines Patienten zu reduzieren, wobei die Menge an Entschäumungsmittel im Bereich von 20 mg bis 150 mg liegt.

2. Formulierung nach Anspruch 1, wobei die Menge an Entschäumungsmittel im Bereich von 35 mg bis 125 mg für die Verwendung nach Anspruch 1 liegt.

3. Formulierung nach Anspruch 1, wobei die Menge an Entschäumungsmittel im Bereich von 3 Gew.-% bis 40 Gew.-% für die Verwendung nach Anspruch 1 liegt.

4. Formulierung nach Anspruch 1, wobei die Menge an Entschäumungsmittel im Bereich von 5 Gew.-% bis 30 Gew.-% für die Verwendung nach Anspruch 1 liegt.

5. Formulierung nach einem der Ansprüche 1 bis 4, wobei es sich bei dem Entschäumungsmittel um mindestens ein Polydimethylsiloxan für die Verwendung nach Anspruch 1 handelt.

6. Formulierung nach Anspruch 5, wobei es sich bei dem Entschäumungsmittel um Dimethicon BP für die Verwendung nach Anspruch 1 handelt.

7. Formulierung nach Anspruch 5, wobei es sich bei dem Entschäumungsmittel um Simethicon BP für die Verwendung nach Anspruch 1 handelt.

8. Formulierung nach Anspruch 5, wobei das Entschäumungsmittel Dimethicon BP und Simethicon BP für die Verwendung nach Anspruch 1 umfasst.

9. Formulierung nach einem der vorhergehenden Ansprüche, wobei die Formulierung zusätzlich ein Antazidum für die Verwendung nach Anspruch 1 enthält.

10. Formulierung nach Anspruch 9, wobei das Antazidum aus der Gruppe ausgewählt ist, die aus einem Carbonatsalz, einem Trisilicat und einem Oxidsalz für die Verwendung nach Anspruch 1 besteht.

11. Formulierung nach einem der vorhergehenden Ansprüche, wobei das Bisphosphonat aus der Gruppe ausgewählt ist, die aus Alendronsäure, Dinatriumetidronat, Dinatriumpamidronat, Ibandronsäure, Risedronatnatrium, Natriumclodronat, Strontiumranelat, Tiludronsäure und Zoledronsäure für die Verwendung nach Anspruch 1 besteht.

12. Formulierung nach einem der vorhergehenden Ansprüche, wobei das Bisphosphonat aus der Gruppe ausgewählt ist, die aus Alendronsäure, Alendronat, Risedronat und Etidronat für die Verwendung nach Anspruch 1 besteht.

13. Formulierung nach einem der vorhergehenden Ansprüche, wobei das Bisphosphonat aus der Gruppe ausgewählt ist, die aus Alendronsäure und Alendronat für die Verwendung nach Anspruch 1 besteht.

14. Formulierung nach einem der vorhergehenden Ansprüche, die zusätzlich (i) ein Vitamin-D-Derivat, (ii) ein Calciumergänzungsmittel oder (iii) sowohl (i) als auch (ii) für die Verwendung nach Anspruch 1 umfasst.

15. Formulierung nach Anspruch 14, wobei das Vitamin-D-Derivat aus der Gruppe ausgewählt ist, die aus Ergocalciferol (Calciferol, Vitamin D2), Cholecalciferol (Vitamin D3), Dihydrotachysterol, Alfacalcidol (1α-Hydroxycholecalciferol) und
Calcitriol (1,25-Dihydroxycholecalciferol) für die Verwendung nach Anspruch 1 besteht.

16. Formulierung nach Anspruch 14, wobei das Calciumergänzungsmittel aus der Gruppe ausgewählt ist, die aus Calciumsalzen, Calciumgluconat, Calciumchlorid, Calciumlactat, ADCAL®, CACIT®, CALCICHEW®, CALCIUM-500®, CALCIUM-SANDOZ® und SANDOCAL® für die Verwendung nach Anspruch 1 besteht.

17. Formulierung nach einem der vorhergehenden Ansprüche, wobei die Formulierung für die gleichzeitige Verabreichung von Wirkstoffen entweder zusammen (in derselben Formulierung) oder separat (gleichzeitig eingenommen) oder separat (zeitlich verzögerte Anwendung) für die Verwendung nach Anspruch 1 formuliert ist.

18. Formulierung nach einem der vorhergehenden Ansprüche, die einen pharmazeutisch geeigneten Träger für die Verwendung nach Anspruch 1 umfasst.

19. Kit, das ein Bisphosphonat für die Verwendung bei der Behandlung von Osteoporose und eine Menge eines Entschäumungsmittels umfasst, die wirksam ist, um Schaumbildung im Magen eines Patienten zu reduzieren, wobei die Menge an Entschäumungsmittel im Bereich von 20 mg bis 150 mg liegt.

20. Kit nach Anspruch 19, wobei die Menge an Entschäumungsmittel im Bereich von 3 Gew.-% bis 20 Gew.-% für die Verwendung nach Anspruch 19 liegt.

21. Kit nach Anspruch 19 oder Anspruch 20, das des Weiteren mindestens ein Vitamin-D-Derivat für die Verwendung nach Anspruch 19 umfasst.

22. Kit nach Anspruch 19 bis 21, das mindestens ein Calciumergänzungsmittel für die Verwendung nach Anspruch 19 umfasst.

23. Verwendung eines Entschäumungsmittels bei der Herstellung eines Medikaments für die Behandlung von oder Vorbeugung gegen Osteoporose in Kombination mit einem Bisphosphonat, wobei das Entschäumungsmittel in einer Menge vorhanden ist, die wirksam ist, um Schaumbildung im Magen eines Patienten zu reduzieren, wobei die Menge an Entschäumungsmittel im Bereich von 20 mg bis 150 mg liegt.

24. Verwendung eines Bisphosphonats bei der Herstellung eines Medikaments für die Behandlung von oder Vorbeugung gegen Osteoporose in Kombination mit einer Menge eines Entschäumungsmittels, die wirksam ist, um Schaumbildung im Magen eines Patienten zu reduzieren, wobei die Menge an Entschäumungsmittel im Bereich von 20 mg bis 150 mg liegt.

25. Verwendung nach Anspruch 23 oder Anspruch 24, wobei die Menge eines Entschäumungsmittels im Bereich von 3 Gew.-% bis 20 Gew.-% liegt.

26. Verwendung nach Anspruch 23 bis 25, wobei das Medikament zusätzlich mindestens ein Vitamin-D-Derivat und Calciumergänzungsmittel enthält.

27. Formulierung für die Verwendung bei der Behandlung von Osteoporose, die Alendronat, eine Menge von Simethicon, die wirksam ist, um die Bildung von Schaum im Magen zu reduzieren, und einen pharmazeutisch geeigneten Träger umfasst, wobei die Menge von Simethicon im Bereich von 20 mg bis 150 mg liegt.

28. Formulierung für die Verwendung bei der Behandlung von Osteoporose, die Alendronat, eine Menge von Dimethicon, die wirksam ist, um die Bildung von Schaum im Magen zu reduzieren, und einen pharmazeutisch geeigneten Träger umfasst, wobei die Menge von Dimethicon im Bereich von 20 mg bis 150 mg liegt.

## Revendications

1. Formulation destinée à être utilisée dans le traitement de l'ostéoporose, comprenant un bisphosphonate en combinaison avec une quantité d'un agent antimousse efficace pour réduire la formation de mousse dans l'estomac d'un patient, dans laquelle la quantité d'agent antimousse se trouve dans la plage de 20 mg à 150 mg.

2. Formulation selon la revendication 1, dans laquelle la quantité d'agent antimousse se trouve dans la plage de 35 mg à 125 mg, destinée à être utilisée selon la revendication 1.

3. Formulation selon la revendication 1, dans laquelle la quantité d'agent antimousse se trouve dans la plage de 3 % à 40 % en poids, destinée à être utilisée selon la revendication 1.

4. Formulation selon la revendication 1, dans laquelle la quantité d'agent antimousse se trouve dans la plage de 5 % à 30 % en poids, destinée à être utilisée selon la revendication 1.

5. Formulation selon l'une quelconque des revendications 1 à 4, dans laquelle l'agent antimousse est un ou plusieurs polydiméthylsiloxanes, destinée à être utilisée selon la revendication 1.

6. Formulation selon la revendication 5, dans laquelle l'agent antimousse est la diméthicone BP, destinée à être utilisée selon la revendication 1.

7. Formulation selon la revendication 5, dans laquelle l'agent antimousse est la siméthicone BP, destinée à être utilisée selon la revendication 1.

8. Formulation selon la revendication 5, dans laquelle l'agent antimousse comprend la diméthicone BP et la siméthicone BP, destinée à être utilisée selon la revendication 1.

9. Formulation selon l'une quelconque des revendications précédentes, dans laquelle la formulation comprend en outre un antiacide, destinée à être utilisée selon la revendication 1.

10. Formulation selon la revendication 9, dans laquelle l'antiacide est choisi dans le groupe constitué d'un sel de carbonate, d'un trisilicate, et d'un sel d'oxyde, destinée à être utilisée selon la revendication 1.

11. Formulation selon l'une quelconque des revendications précédentes, dans laquelle le bisphosphonate est choisi dans le groupe constitué de l'acide alendronique, de l'étidronate disodique, du pamidronate disodique, de l'acide ibandronique, du risédronate de sodium, du clodronate de sodium, du ranélate de strontium, de l'acide tiludronique et de l'acide zolédronique, destinée à être utilisée selon la revendication 1.

12. Formulation selon l'une quelconque des revendications précédentes, dans laquelle le bisphosphonate est choisi dans le groupe constitué de l'acide alendronique, de l'alendronate, du risédronate et de l'étidronate, destinée à être utilisée selon la revendication 1.

13. Formulation selon l'une quelconque des revendications précédentes, dans laquelle le bisphosphonate est choisi dans le groupe constitué de l'acide alendronique et de l'alendronate, destinée à être utilisée selon la revendication 1.

14. Formulation selon l'une quelconque des revendications précédentes, comprenant en outre (i) un dérivé de la vitamine D, (ii) un supplément de calcium ou (iii) à la fois (i) et (ii), destinée à être utilisée selon la revendication 1.

15. Formulation selon la revendication 14, dans laquelle le dérivé de vitamine D est choisi dans le groupe constitué de l'ergocalciférol (calciférol, vitamine D2), du cholécalciférol (vitamine D3), du dihydrotachystérol, de l'alfacalcidol (1α-hydroxycholécalciferol), et du calcitriol (1,25-dihydroxycholécalciferol), destinée à être utilisée selon la revendication 1.

16. Formulation selon la revendication 14, dans laquelle le supplément de calcium est choisi dans le groupe constitué des sels de calcium, du gluconate de calcium, du chlorure de calcium, du lactate de calcium, d'ADCAL®, de CACIT®, de CALCICHEW®, de CALCIUM-500®, de CALCIUM SANDOZ® et de SANDOCAL®, destinée à être utilisée selon la revendication 1.

17. Formulation selon l'une quelconque des revendications précédentes, dans laquelle la formulation est formulée pour une administration simultanée des principes actifs, soit ensemble (dans la même formulation) soit séparément (pris ensemble au même moment), ou séparément (administration retardée), destinée à être utilisée selon la revendication 1.

18. Formulation selon l'une quelconque des revendications précédentes, comprenant un vecteur pharmaceutiquement acceptable, destinée à être utilisée selon la revendication 1.

19. Kit comprenant un bisphosphonate destiné à être utilisé dans le traitement de l'ostéoporose et une quantité d'un agent antimousse efficace pour réduire la formation de mousse dans l'estomac d'un patient, dans lequel la quantité d'agent antimousse se trouve dans la plage de 20 mg à 150 mg.

20. Kit selon la revendication 19, dans lequel la quantité d'agent antimousse se trouve dans la plage de 3 % à 20 % en poids, destiné à être utilisé selon la revendication 19.

21. Kit selon la revendication 19 ou la revendication 20, comprenant en outre un ou plusieurs dérivés de la vitamine D, destiné à être utilisé selon la revendication 19.

22. Kit selon les revendications 19 à 21, comprenant un ou plusieurs suppléments du calcium, destiné à être utilisé selon la revendication 19.

23. Utilisation d'un agent antimousse dans la fabrication d'un médicament destiné au traitement ou à la prophylaxie de l'ostéoporose en combinaison avec un bisphosphonate, dans laquelle l'agent antimousse est présent en une quantité efficace pour réduire la formation de mousse dans l'estomac d'un patient, et dans laquelle la quantité d'agent antimousse se trouve dans la plage de 20 mg à 150 mg.

24. Utilisation d'un bisphosphonate dans la fabrication d'un médicament destiné au traitement ou à la prophylaxie de l'ostéoporose en combinaison avec une quantité d'un agent antimousse efficace pour réduire la formation de mousse dans l'estomac d'un patient, dans laquelle la quantité d'agent antimousse se trouve dans la plage de 20 mg à 150 mg.

25. Utilisation selon la revendication 23 ou la revendication 24, dans laquelle la quantité d'agent antimousse se trouve dans la plage de 3 % à 20 % en poids.

26. Utilisation selon les revendications 23 à 25, dans laquelle le médicament supplémentaire comprend un ou plusieurs dérivés de la vitamine D et des suppléments du calcium.

27. Formulation destinée à être utilisée dans le traitement de l'ostéoporose, comprenant de l'alendronate, une quantité de siméthicone efficace pour réduire la formation de mousse dans l'estomac, et un vecteur pharmaceutiquement acceptable, dans laquelle la quantité de siméthicone se trouve dans la plage de 20 mg à 150 mg.

28. Formulation destinée à être utilisée dans le traitement de l'ostéoporose, comprenant de l'alendronate, une quantité de diméthicone efficace pour réduire la formation de mousse dans l'estomac, et un vecteur pharmaceutiquement acceptable, dans laquelle la quantité de diméthicone se trouve dans la plage de 20 mg à 150 mg.
